# EUROPEAN PATENT APPLICATION

(11) **EP 4 803 630 A1**
(43) Date of publication of application: **09.09.2026**
(21) Application number: 24885760.9
(22) Date of filing: 30.10.2024
(51) Int. Cl.: C12N 15/85, C12N 5/10, C12N 15/12, C12N 15/53, C12N 15/56, C12Q 1/02, C12Q 1/34, C12Q 1/66, C12Q 1/6897, G01N 33/15, G01N 33/50

(54) **CELL FOR IDENTIFYING MAIT CELL LIGAND, AND METHOD FOR SCREENING MAIT CELL LIGAND USING SAID CELL**

(30) Priority: 31.10.2023 JP 2023186971
(71) Applicant: The University of Osaka, Osaka 565-0871 (JP)
(72) Inventor: YAMASAKI, Sho, Suita-shi, Osaka 565-0871 (JP); ITO, Emi, Suita-shi, Osaka 565-0871 (JP)
(74) Representative: Weickmann & Weickmann PartmbB
(86) International application number: PCT/JP2024/038691
(87) International publication number: WO 2025/094993

(57) **Abstract**

The present specification discloses, as embodiments, T cells that co-express MR1 molecules and TCR of MAIT cells, and that express a reporter protein in response to activation of the TCR of MAIT cells, a method for screening for ligands for MAIT cells, by using the T cells, and the like.

## Description

### [Technical Field]

The present invention provides, as embodiments thereof, cells for identifying ligands for Mucosal-associated invariant T (MAIT) cells, and methods for screening MAIT cell ligands using the cells, and is useful, for example, in the pharmaceutical field.

### [Background Art]

MAIT cells, classified as innate immune T cells, are the largest T cell population accounting for several percent of human T cells, and recognize bacterial metabolites presented on the MHC-related-1 (MR1) molecule as antigens. 5-(2-Oxopropylidene-amino)-6-D-ribitylaminouracil (5-OP-RU), which is said antigen, is an intermediate of riboflavin metabolites found in many bacteria, and is a system capable of inducing an immune response against a broad spectrum of bacteria (Non Patent Literature 1).

### [Citation List]

### [Non Patent Literature]

[Non Patent Literature 1]
   Corbett, Nature 2014
[Non Patent Literature 2]
   Awad, Immunol. Cell Biol. 2018
[Non Patent Literature 3]
   Constantinides, Science 2019
[Non Patent Literature 4]
   Legoux, Science 2019
[Non Patent Literature 5]
   Lepore, eLife 2017
[Non Patent Literature 6]
   Crowther, Nat. Immunol. 2020

### [Summary of Invention]

### [Technical Problem]

However, the above-mentioned antigen only binds to a portion of the vast MR1 molecular pocket (Non Patent Literature 2), and the existence of other unidentified antigens has been suggested. While the above-mentioned antigen is considered to play a role in the thymic differentiation of MAIT cells (Non Patent Literatures 3 and 4), the existence of endogenous antigens in MAIT cells has also been suggested from a different perspective (Non Patent Literatures 5 and 6). Therefore, there was a strong need to create a screening method that could efficiently search for and identify MAIT cell ligands.

### [Solution to Problem]

MR1 molecules are present in almost all cells in the body, but under steady state, only a small portion is expressed on the T cell surface, and most of them are localized within the intracellular endoplasmic reticulum. However, under infection state, etc., MR1 molecules presenting their antigen (e.g., 5-OP-RU) migrate to the T cell surface and are expressed, thus presenting the antigen to MAIT cells and inducing activation of MAIT cells.

The present inventors reached a novel finding that, if it is possible to simultaneously evaluate, using a single cell, both 1) the expression of the MR1 molecule on the T cell surface due to the binding of a test substance to the MR1 molecule, and 2) the subsequent induction of activation of the TCR of MAIT cells, then a new MAIT cell antigen (activating ligand) can be efficiently searched for and identified. They have conducted intensive studies and completed the present invention.

Specific embodiments of the present invention are as follows. However, the present invention is not limited to these.
[1] A T cell that co-expresses an MR1 molecule and a TCR of a MAIT cell and expresses a reporter protein in response to a stimulation of the TCR.
[2] The T cell of the above-mentioned [1], wherein the reporter protein is selected from green fluorescent protein (GFP), luciferase, and β-glucuronidase.
[3] The T cell of the above-mentioned [1], wherein the reporter protein is GFP.
[4] The T cell of any of the above-mentioned [1] to [3], wherein the MR1 molecule and the TCR of the MAIT cell are derived from human.
[5] A method for screening for a MAIT cell agonist or antagonist, comprising at least the following steps:
   (1) a step of contacting the T cell according to any one of the above-mentioned [1] to [4] with a test substance, and
   (2) a step of observing the activation of a TCR of a MAIT cell and the expression of an MR1 molecule on the T cell surface to
      (i) determine a test substance that activates the TCR of the MAIT cell as an agonist of the MAIT cell, and to
      (ii) determine a test substance that enhances the expression of the MR1 molecule on the T cell surface without activating the TCR of the MAIT cell as an antagonist of the MAIT cell.
[6] The screening method of the above-mentioned [5], wherein the activation of the TCR of the MAIT cell is determined by the intensity of GFP emission.
[7] The screening method of the above-mentioned [5] or [6], wherein the activation of the TCR of the MAIT cell and the expression of the MR1 molecule on the T cell surface are observed by flow cytometry.
[8] The screening method of any of the above-mentioned [5] to [7], wherein the test substance is a compound group sample.
[9] A biomarker for testing for an inflammatory disease caused by cholestasis, comprising CA3S (cholic acid 3-sulfate) or CA7S (cholic acid 7-sulfate).
[10] The biomarker of the above-mentioned [9], wherein the inflammatory disease caused by cholestasis is primary biliary cholangitis (PBC) or primary sclerosing cholangitis (PSC).
[11] A method for testing for an inflammatory disease caused by cholestasis, comprising measuring a concentration of CA3S or CA7S in bile collected from a test subject.

### [Advantageous Effects of Invention]

The present invention provides, as embodiments thereof, cells for identifying MAIT cell ligand with high sensitivity, and efficient methods for screening for MAIT cell ligands using the cells. In another embodiment, a biomarker for testing for inflammatory diseases caused by cholestasis is provided.

### [Brief Description of Drawings]

[Fig. 1]

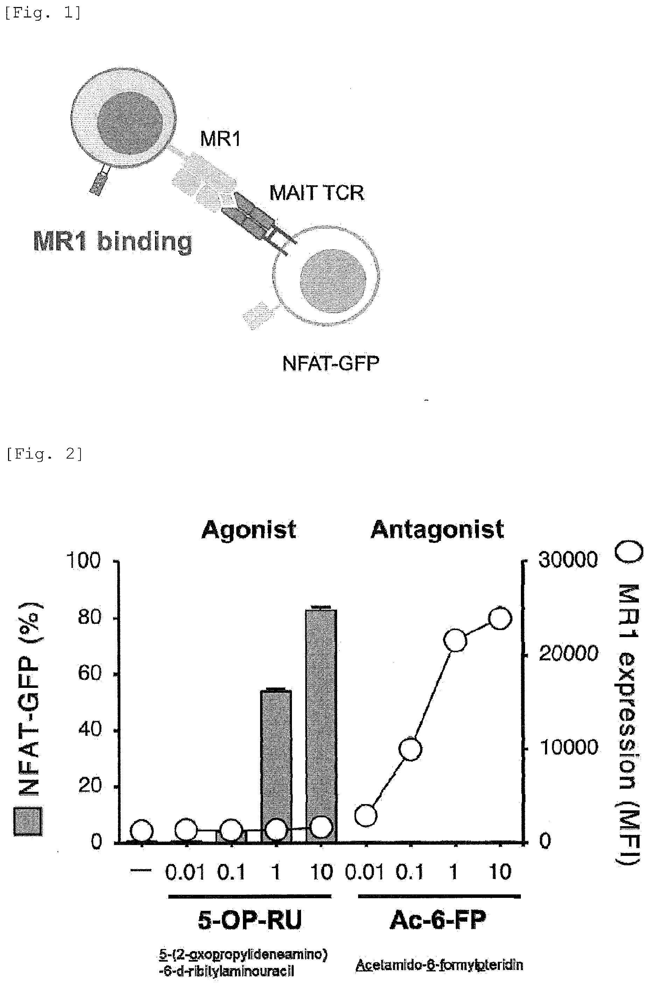

   Fig. 1 shows a conceptual diagram of reporter cells ("co-expression reporter cells") co-expressing MR1 molecule and TCR of MAIT cell (Example 1 described below).
[Fig. 2]
   Fig. 2 shows the validation results of the co-expression reporter cells as a screening system using existing MAIT cell ligands (Example 1 described below).
[Fig. 3]

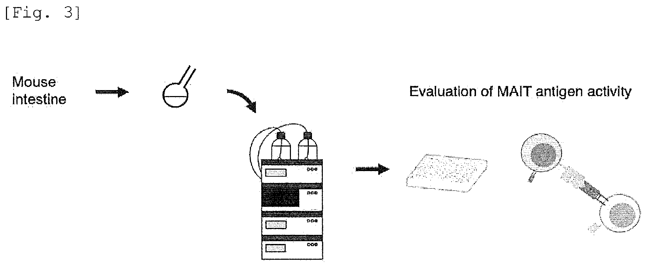

   Fig. 3 shows a conceptual diagram of a method for screening for MAIT cell ligand by using co-expression reporter cell (Example 2 described below).
[Fig. 4]

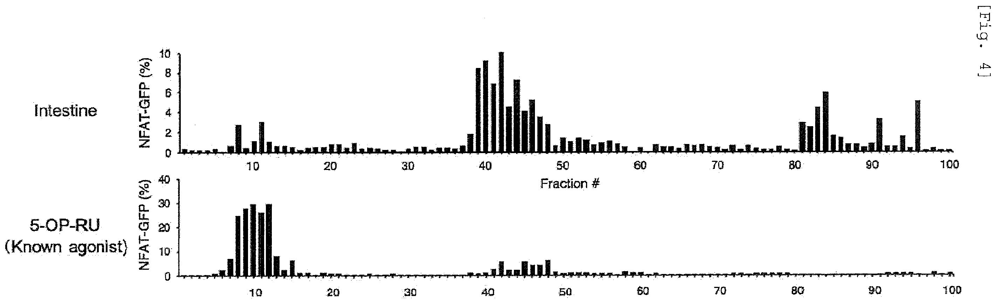

   Fig. 4 shows the evaluation results of reporter cells for each fraction obtained by separating the intestinal extract and 5-OP-RU of SPF mice by reverse-phase column chromatography (Example 2 described below).
[Fig. 5]

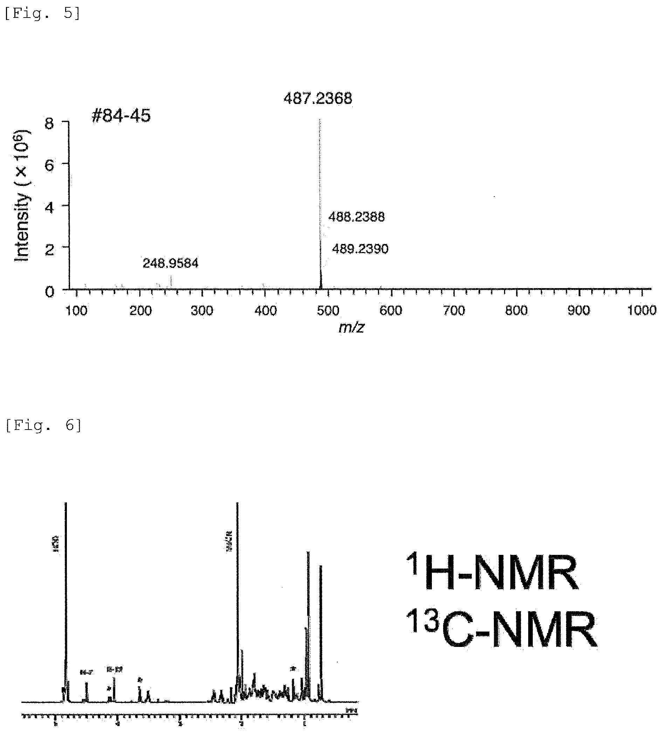

   Fig. 5 shows the evaluation results of the activation molecule of fraction #84-45 by high-resolution mass spectrometry (HRMS) (Example 2 described below).
[Fig. 6]
   Fig. 6 shows the results of NMR spectroscopy analysis of the activation molecule of fraction #84-45 (Example 2 described below).
[Fig. 7]

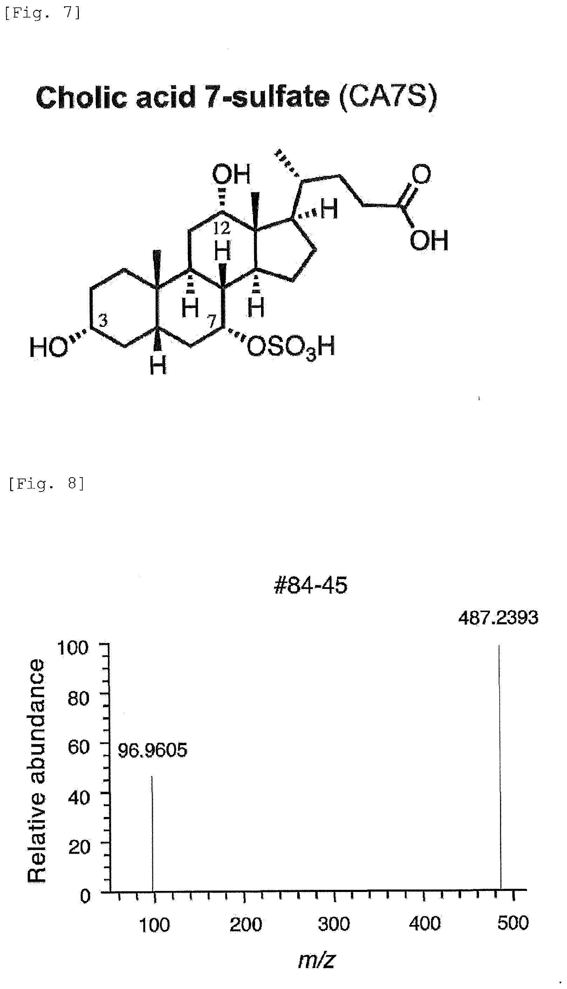

   Fig. 7 shows the structure of cholic acid 7-sulfate (CA7S).
[Fig. 8]
   Fig. 8 shows the confirmation results of HRMS/MS that the activation molecule of fraction #84-45 has a sulfate group (Example 2 described below).
[Fig. 9]

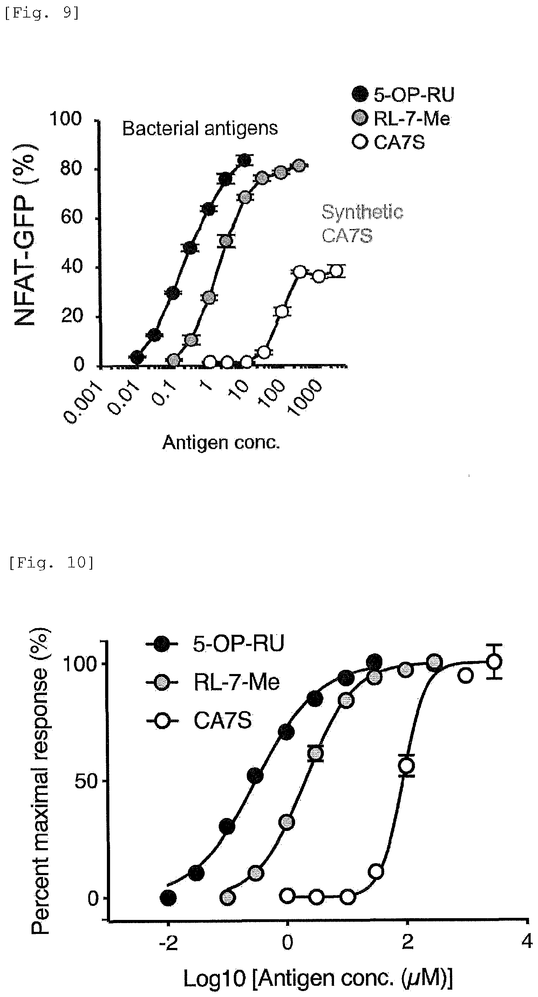

   Fig. 9 shows the evaluation results of CA7S by co-expression reporter cell (Example 2 described below).
[Fig. 10]
   Fig. 10 shows a comparison of the agonist activity of CA7S and ribitylrumazine (RL-7-Me) against MAIT cell, demonstrating that CA7S is a weak agonist against MAIT cell (Example 2 described below).
[Fig. 11]

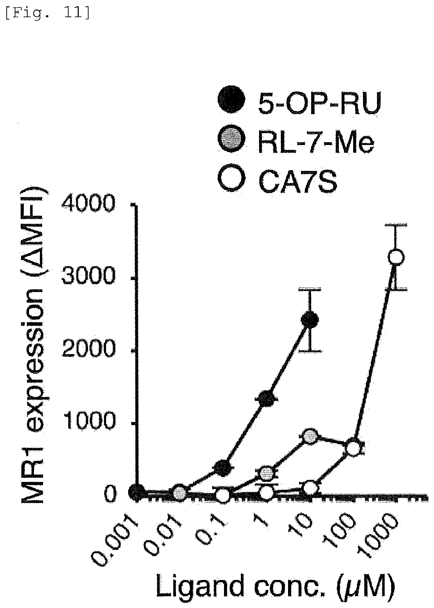

   Fig. 11 shows that CA7S increased the expression of MR1 on the T cell surface (Example 2 described below).
[Fig. 12]

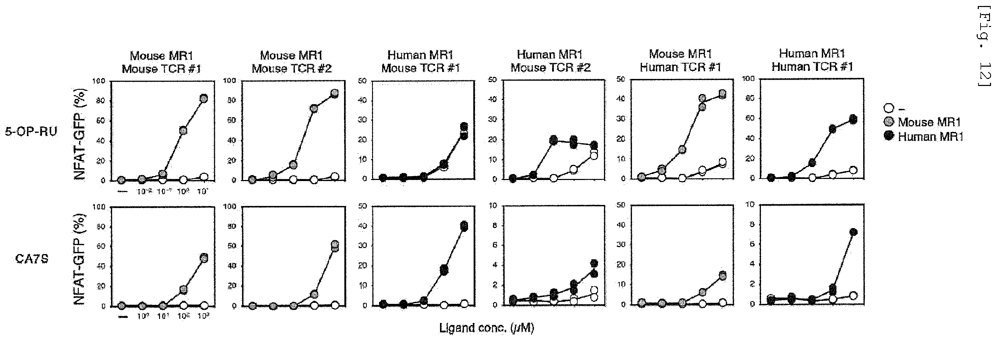

   Fig. 12 shows that the activity of CA7S was confirmed in the TCR of MAIT cells derived from mouse (m) or human (h) in the presence of mouse or human MR1 (Example 2 described below).
[Fig. 13]

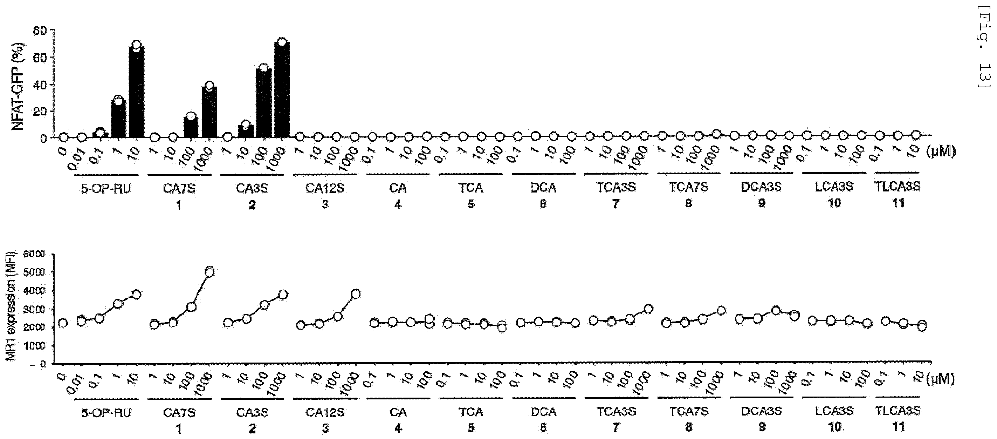

   Fig. 13 shows the study results of Example 3 described below, that cholic acid (CA) did not increase the expression of either TCR of MAIT cells or MR1 on the T cell surface (Fig. 13A and B), CA7S and CA3S activated TCR of MAIT cells, but CA12S did not (Fig. 13, upper), and CA7S, CA3S, and CA12S all increased the expression of MR1 molecules on the T cell surface (Fig. 13, lower).
[Fig. 14]

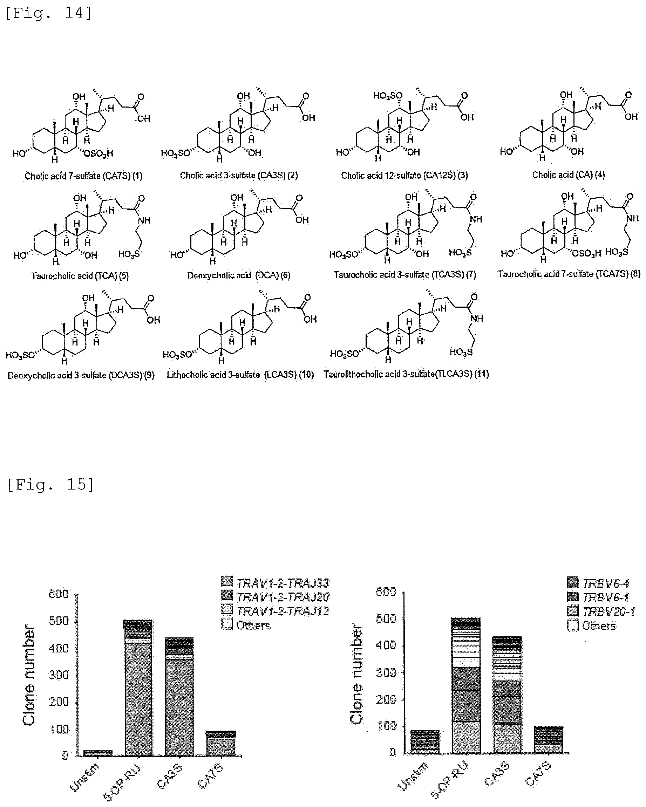

   Fig. 14 shows the structures of CA7S and its analogs studied in the Example.
[Fig. 15]
   Fig. 15 shows the results of sc-TCR-RNA-seq analysis after long-term stimulation with CA7S, CA3S, and 5-OP-RU (Example 4 described below).
[Fig. 16]

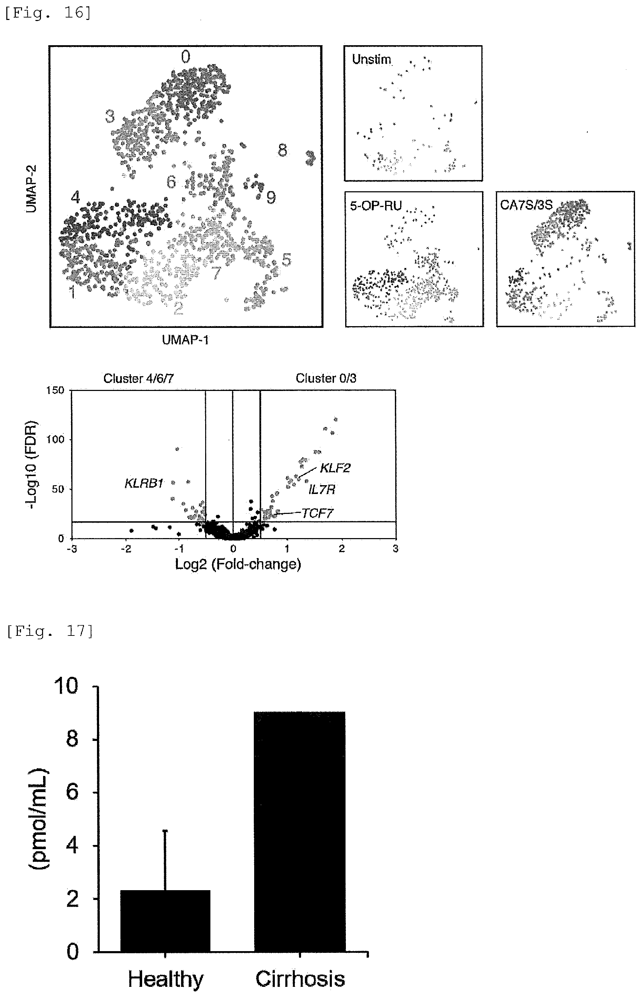

   Fig. 16 shows the results of sc-TCR-RNA-seq analysis after long-term stimulation with CA7S, CA3S, and 5-OP-RU (Example 4 described below).
[Fig. 17]
   Fig. 17 shows the evaluation results of CA3S concentration (pmol/ml) in bile of subjects in a healthy state and a state of cholestasis. It shows that the CA3S concentration is higher in the state of cholestasis compared to the healthy state.
[Fig. 18]

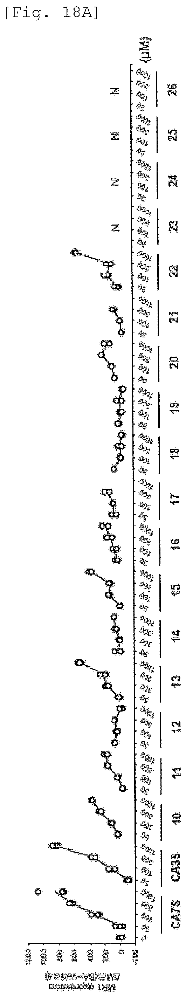

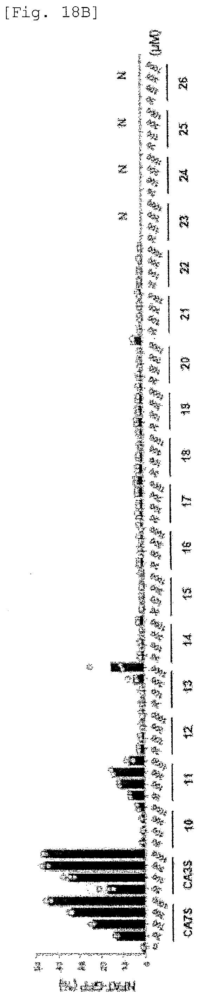

   Fig. 18 shows the study results of the activation effect of CA7S and its analogs on TCR of MAIT cells, and the activation effect thereof on the expression of MR1 molecules on the T cell surface as studied in Example 6 described below.
[Fig. 19]

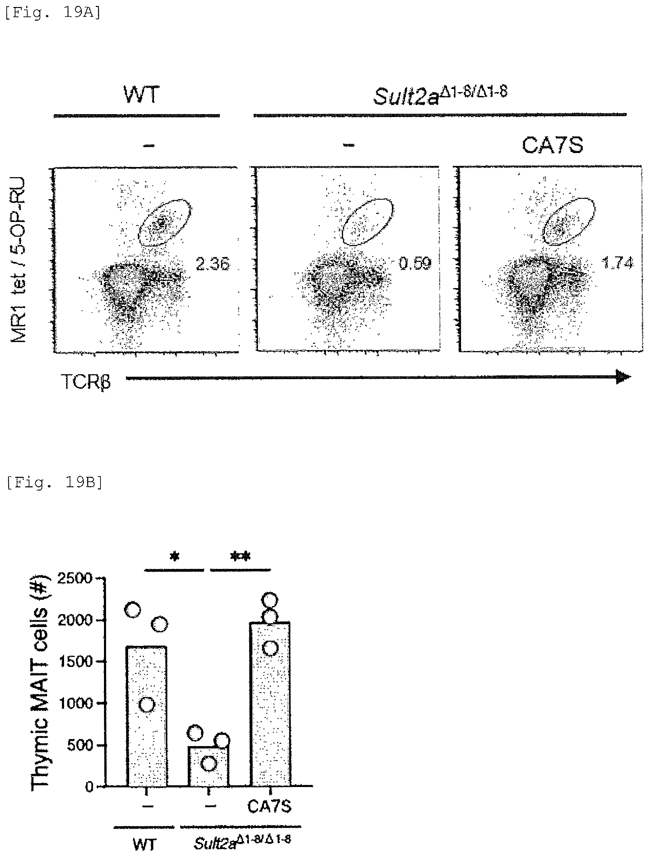

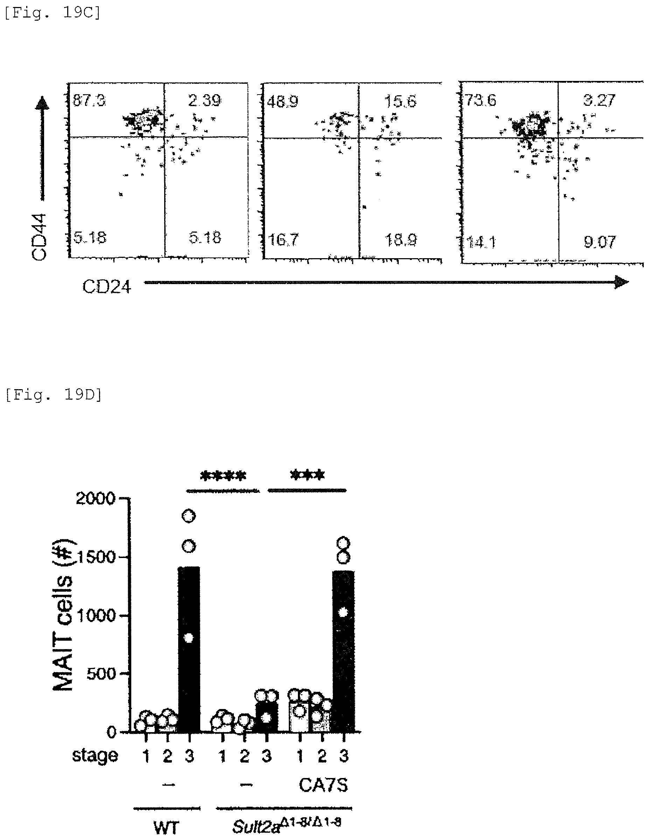

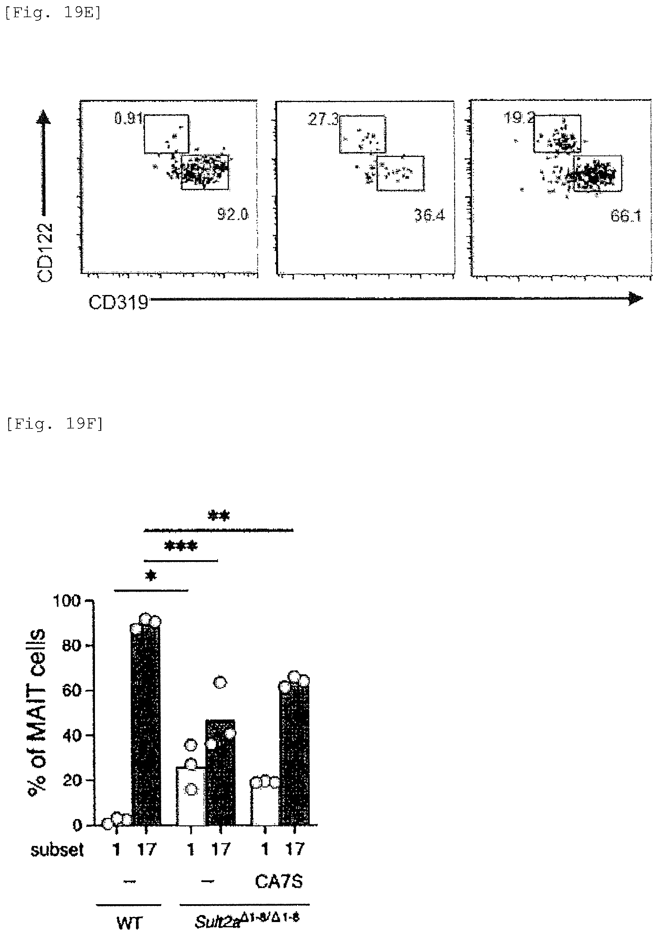

   Fig. 19 shows the results of flow cytometry analysis of MAIT cell expression in MR1-5-OP-RU tetramer-enriched thymocytes in Sult2a-deficient mice, with and without CA7S administration.

### [Description of Embodiments]

In the following, the present invention is described with reference to specific embodiments as examples. Unless otherwise specified, all technical and scientific terms used in the present specification have the same meaning as generally understood by one of ordinary skill in the art to which the present invention belongs.

### [Co-expression reporter cell]

One embodiment of the present invention is a "T cell that co-expresses an MR1 molecule and a TCR of a MAIT cell and expresses a reporter protein in response to the activation of the TCR of the MAIT cell" (hereinafter also referred to as a "co-expression reporter cell").

Since the above-mentioned "co-expression reporter cell" co-expresses the MR1 molecule and the TCR of MAIT cells, it is possible to simultaneously evaluate, using a single cell, both 1) the expression of the MR1 molecule on the T cell surface due to the binding of a test substance to the MR1 molecule, and 2) the subsequent induction of activation of the TCR of MAIT cells. Since MAIT cells are activated through the activation of TCR thereof, it has become possible to efficiently search for and identify MAIT cell activation ligands based on the evaluation.

### (Cell, etc.)

The T cells used in this embodiment are not particularly limited as long as they can co-express the MR1 molecule and the TCR of MAIT cells, and may be human-derived T cells or animal-derived (e.g., mouse-derived) T cells.

Furthermore, the MR1 molecule and the TCR of MAIT cells used in this embodiment are not particularly limited as long as they can co-express, and may be human-derived or animal-derived (e.g., mouse-derived). From the viewpoint of clinical application, those derived from human are preferred.

### (Reporter protein)

A preferred embodiment of the above-mentioned "co-expression reporter cell" is one that expresses a reporter protein in response to the activation of the TCR of MAIT cells, in order to efficiently and highly sensitively detect the activation of the TCR of MAIT cells.

The reporter protein to be expressed is not particularly limited as long as the expression thereof can be easily observed, and proteins widely used in this technique field can be used. For example, proteins widely used in this technique field whose expression can be easily observed, such as green fluorescent protein (GFP), luciferase, β-glucuronidase, and the like can be used. Among these, proteins whose expression level can be observed by the intensity of the luminescence are preferred, and GFP is particularly preferred.

The above-mentioned reporter protein needs to be expressed in response to the activation of the TCR of MAIT cells. For example, a system in which the reporter protein is expressed downstream of the TCR activation signal can be used.

### (Creation of co-expression reporter cells)

The above-mentioned "co-expression reporter cell" can be created by a person skilled in the art by preparing a T cell in which the MR1 molecule gene, the TCR gene of MAIT cells, and a gene that expresses a reporter protein in response to a stimulation of the TCR have been co-introduced, using, for example, genetic engineering techniques generally used in the art (e.g., genetic recombination technique, gene transfer technique, and the like). For example, a person skilled in the art can appropriately create them by referring to the method described in Example 1 below.

### [Method for screening for MAIT cell ligand]

Another embodiment of the present invention is a method for screening for a MAIT cell agonist or antagonist. Specifically, it is "a method for screening for a MAIT cell agonist or antagonist, including at least the following steps:
(1) a step of contacting the above-mentioned "co-expression reporter cell" with a test substance, and
(2) a step of observing the activation of a TCR of a MAIT cell and the expression of an MR1 molecule on the T cell surface to
   (i) determine a test substance that activates the TCR of the MAIT cell as an agonist of the MAIT cell and to
   (ii) determine a test substance that enhances the expression of the MR1 molecule without activating the TCR of the MAIT cell as an antagonist of the MAIT cell.

### (Determination of being ligand)

In the screening method of this embodiment, where a test substance activates TCR of MAIT cells, the test substance is determined to be an agonist of MAIT cells (step (2) (i)). Furthermore, where a test substance promotes expression of MR1 molecules on the T cell surface, the test substance is considered to be an agonist with stronger binding to the MR1 molecule.

In this determination, for example, where the level of TCR activation (e.g., NFAT-GFP (%)) by stimulation with a test substance is three times or more higher than the activation level without the stimulation, the test substance can be determined to be an agonist.

In the screening method of this embodiment, where a test substance enhances the expression of MR1 molecules on the T cell surface but does not activate TCR of MAIT cells, the test substance is determined to be an antagonist of MAIT cells (step (2) (ii)).

In this determination, for example, where the expression level of MR1 molecule (e.g., MFI value) when stimulated with a test substance is three times or more higher than the level without stimulation, the test substance can be determined to be an antagonist.

As described above, a superior feature of a screening method using the present "co-expression reporter cell" is that it can search for and identify not only agonists but also antagonists of MAIT cells.

In the above-mentioned determination, the expression level of TCR of MAIT cells can be determined, for example, by evaluating the intensity of reporter protein luminescence using flow cytometry.

Furthermore, the expression level of MR1 molecules on the T cell surface can be determined, for example, by performing fluorescent antibody staining, followed by evaluation using flow cytometry.

In the present screening, it is preferable that the expression level of TCR of MAIT cells and the expression level of MR1 molecules on the T cell surface be determined using the same evaluation method. Therefore, it is preferable to determine the reporter protein expression system also taking this view point into consideration.

### (Test substance)

The test substance evaluated in the screening method of this embodiment may be an isolated and purified single compound sample, or a compound group sample consisting of a number of single compounds. For example, the sample may contain a vast number of compounds to be evaluated, such as extracts from tissues of organs and the like or a library of endogenous metabolites.

When using a "single compound sample," it is possible to directly determine whether the test substance is an agonist or antagonist of MAIT cells.

When using a "compound group sample," it is possible to first determine whether candidate compounds that could act as MAIT cell agonists or antagonists are present in the compound group. Then, using methods generally used in this technique field, the candidate compounds can be narrowed down and the structures thereof can be determined by purification and isolation.

When using a "compound group sample," it is preferable to first separate the samples into a predetermined number of fractions using an appropriate purification method (e.g., HPLC), and then subject each fraction to evaluation using this screening method. By combining this with prior purification of the evaluation sample, it is possible to efficiently and sensitively search for and identify MAIT cell agonists or antagonists from a "compound group sample" containing a large number of compounds. In particular, when using an endogenous metabolite group, it is expected that endogenous agonists and antagonists can be identified, leading to effective application to drug discovery.

More specifically, the method shown in Example 2 described below can be referred to in the search for agonists in MAIT cells by using the above-mentioned "compound group sample." Each fraction is evaluated using "co-expression reporter cell" and a fraction containing the test substance that activates TCR of MAIT cells is selected. Candidate compounds are identified from this fraction, the identified compounds are further evaluated using the "co-expression reporter cell" and confirmed to be agonists according to the above-mentioned criteria, whereby the desired agonists can be obtained.

Furthermore, it is also possible to synthesize or purchase derivatives of the compounds identified as agonists or antagonists in the above, and further evaluate the derivatives by the present screening method to further obtain other agonists or antagonists.

### (Contact between test substance and co-expression reporter cell)

The conditions (temperature, time, specific procedure) for stimulating co-expression reporter cells by contact between a test substance and co-expression reporter cells can be appropriately set by those skilled in the art depending on the test substance to be evaluated and the like. Simply put, this can be done, for example, by culturing "co-expression reporter cells" on a suitable medium in each well of a 96-well plate and adding the test substance. The test substance may be in a solution state using a suitable solvent (water, alcohol, etc.) where necessary.

Contact can be performed, for example, within a temperature range of 25 to 45°C. After contact for, for example, 1 to 24 hr, the activation status of TCR of MAIT cells and the expression of MR1 molecules on the T cell surface can be observed to determine agonist activity or antagonist activity.

### [Bile acid metabolites]

As described in Examples 2 and 3 below, the present inventors, using the above-mentioned screening method, searched for MAIT cell ligands from mouse intestinal extracts containing a vast number of metabolites. As a result, they identified for the first time that CA7S and CA3S, which constitute bile acid metabolites that are major components of intestinal secretions from the gallbladder, are agonists of MAIT cells. This fact clearly demonstrates the usefulness of the above-mentioned screening method.

As shown in Example 3 below, other analogs with different sulfation positions did not exhibit agonist action on MAIT cells, and it was confirmed that the action is specific to CA7S and CA3S.

Furthermore, as shown in Example 4 below, it was also revealed that CA7S and CA3S, unlike the conventional exogenous agonist 5-OP-RU, have the characteristic of contributing to the development and survival of MAIT cells.

Conventionally, no endogenous MAIT cell agonists were known, and the agonist newly found this time is highly expected to be utilizable in future drug discovery research and clinical applications.

### [CA3S and cholestasis]

Cholic acid sulfate (CAS) is derived from cholic acid (CA) and is sulfated by the sulfotransferase 2a (Sult2a). In mice, there are eight isoforms of Sult2a (2a1-2a8), and Sult2a8, which is particularly highly expressed, attaches a sulfate group at the 7th position of CA, and is thus abundant in CA7S. In contrast, humans express only SULT2A1, which attaches a sulfate group at the 3rd position, and are thus almost exclusively CA3S.

Therefore, of CA7S and CA3S, which were newly found to be MAIT cell agonists, CA3S was considered more suitable for application to humans in a medical setting, and the application of CA3S in a medical setting was further investigated.

With respect to inflammatory diseases associated with cholestasis, it has been reported that (1) there is a correlation between primary sclerosing cholangitis (PSC) and primary biliary cholangitis (PBC), and the activation of MAIT cells (Am. J. Pathol. 987, 192, 629-641 (2022); J. Autoimmun. 90, 64-75 (2018)), (2) MAIT cells are activated in the livers of PBC and PSC patients (J. Autoimmun. 2018; Eur. J. Immunol. 2018), and (3) bile from PBC patients has been shown to activate MAIT cells (Am. J. Pathol. 2022).

However, while excessive activation of MAIT cells is suspected to be the cause of the disease, it remains unclear which components (molecular entities) in bile activated MAIT cells.

Based on the novel finding that CA3S is an endogenous agonist of MAIT cells, the present inventors conceived a novel idea that CA3S could be used as a diagnostic marker for the onset of cholestasis. They compared the concentration of CA3S in bile (pmol/ml) in healthy humans and in humans who developed cholestasis. As shown in Example 5 below, it was confirmed that the concentration of CA3S increased to about four times that of healthy humans after the onset of cholestasis.

As described above, it has become clear that CA3S can be used as a biomarker for the examination (diagnosis) of the onset of cholestasis. Therefore, CA3S can also be used for the examination (diagnosis) of inflammatory diseases caused by cholestasis. When used as a biomarker, CA3S can be used in either the form of CA3S alone or as a composition containing a carrier generally used in the pharmaceutical field. Those skilled in the art can appropriately select the form thereof according to the purpose.

Such an examination (diagnosis) can be performed, for example, by measuring the concentration of CA3S in bile, collected from a patient, using equipment generally used in the technical field (e.g., mass spectrometer). Where the measured value is significantly elevated compared to a healthy state, a diagnosis of possible inflammatory disease can be made.

Furthermore, CA7S is also expected to be useful as a biomarker in a similar manner.

### [Example]

The present invention is described in detail below based on Examples, but the present invention is not limited thereto. Those skilled in the art can modify embodiments of the present invention in various embodiments without departing from the meaning of the present invention, and such modifications are also included within the scope of the present invention.

### Example 1: Creation of T cells co-expressing MR1 molecule and MAIT cell TCR in combination with reporter assay and verification as screening system

### (1) Creation of co-expressing T cells

MAIT TCR genes and MR1 molecules genes were synthesized from mouse (Tiloy et al, J Exp Med. 1999) or human (4L4T, Protein Data Bank) and inserted into retroviral vectors (pMX-IRES-ratCD2, pMX-IRES-humanCD8), respectively. These plasmids were co-introduced into Phoenix packaging cells by using PEI MAX (Polysciences). TCR-deficient mouse T cell hybridomas (Matsumoto, BBRC.2020) possessing the NFAT-GFP reporter gene were infected with a supernatant containing retrovirus. MAIT TCR and MR1 co-expressing T cells (hereinafter also referred to as "co-expression reporter cells") were selected using antimouse TCRβ (H57-597), anti-human CD3 (HIT3a), and anti-human/mouse/rat MR1 (26.5) antibodies.

Fig. 1 shows a conceptual diagram of these "co-expression reporter cells."

### (2) Validation as screening system

Using 5-OP-RU, known as an antigen for MAIT cells, and acetyl-6-formylpterin (Ac-6-FP), known as a non-irritating MR1 molecular ligand, it was verified that the "co-expression reporter cells" created in (1) could be used as a screening system for MAIT cell ligands.

5-OP-RU was prepared using 5-A-RU (Toronto Research Chemical) and methylglyoxal (Sigma-Aldrich) according to a previous report (Nature. 509, 361-365 (2014)). Ac-6-FP (Cat. No. 11.418) was purchased from Schricks Laboratories.

The validation experiment evaluated GFP expression and MR1 molecule surface expression 6 hr after stimulation of co-expression reporter cells with vehicle control, 5-OP-RU, and Ac-6-FP. GFP was evaluated by flow cytometry. MR1 molecule surface expression was represented by the MFI value of the stimulated cells.

As shown in Fig. 2, 5-OP-RU dose-dependently enhanced GFP expression. On the other hand, Ac-6-FP strongly increased MR1 molecule expression on the T cell surface without enhancing GFP expression. Therefore, it was verified that the above-mentioned "co-expression reporter cells" can be used to evaluate the agonist action or antagonist action of a test substance on MAIT cells.

### Example 2: Screening of MAIT cell ligands from mouse intestinal extracts

(1) To screen for host-derived ligands for MAIT cells, fractional extracts from the intestines of pathogen-free (SPF) mice (CREA Japan) were screened using a "co-expression reporter cell" line. Fig. 3 shows a conceptual diagram of the screening for MAIT cell ligands in this Example.

In the 100 fractions obtained by separating the extract from the mouse intestines by reverse-phase column chromatography, three large peaks were detected around fractions #11, #42, and #84 (Fig. 4, upper diagram). To investigate which peaks correspond to known antigens derived from microorganisms, 5-OP-RU was subjected to the same column separation process. The activity was again observed in fractions #11 and #42 (Fig. 4, lower diagram), which corresponded to the expected retention times of 5-OP-RU and its related lumazine derivatives, respectively.

In the above-mentioned screening, to stimulate "co-expression reporter cells," tissue extract samples and synthetic compounds were dissolved in water and added to each well of a 96-well plate in the absence of methylglyoxal. Some water-insoluble samples were dissolved in ethanol or chloroform/methanol (2:1, vol/vol) and coated onto the plates. "Co-expression reporter cells" (1-3×10⁴ cells/100 µL/well) were cultured at 37°C for 6-20 hr, and the expression of GFP and MR1 molecules was analyzed by flow cytometry. The same procedure was performed in the subsequent Examples.
(2) To determine the chemical structure of the activation molecule contained in fraction #84, this fraction was further purified by hydrophilic interaction chromatography (HILIC), and a single fraction (named #84-45) was recovered. This activity was resistant to nucleases and proteases, suggesting that it is a metabolite other than polypeptides or oligonucleotides. High-resolution mass spectrometry (HRMS) detected a major m/z peak at 487.2368 in negative ion mode, indicating that it is a low-molecular-weight compound that readily undergoes negative ionization (Fig. 5).
(3) Next, 1D and 2D-NMR spectroscopy was performed. The results are shown in Fig. 6. The 1H and 13C NMR spectra showed signals characteristic of cholic acid analogs (in the 1H NMR spectrum, peaks of three methyl protons at δ0.73, 0.93, and 0.97 ppm, two methylene protons at δ2.32 and 2.45 ppm, and three methine protons at δ3.52, 4.07, and 4.51 ppm; in the 13C NMR spectrum, a peak of carbonyl carbon at δ180.8 ppm). Extensive analysis, including 2D-NMR, identified two peaks in the 1H NMR spectrum at δ 4.07 and 4.51 ppm as methine protons H-12 and H-7, respectively. The H-7 signal appeared more deshielded than predicted, indicating that the hydroxyl group at the C7 position of the cholic acid skeleton was bonded to the functional group. Furthermore, the molecular formula estimated from the main peak at m/z 487.2368 was C₂₄H₄₀O₈S[M-H], suggesting that the main component of this fraction is cholic acid 7-sulfate (CA7S) (Fig. 7). Indeed, the presence of the sulfate group was confirmed by HRMS/MS analysis (Fig. 8).
(4) To confirm the above-mentioned presumed structure, CA7S was synthesized according to a previous report (Chem. Pharma. Bull. 27, 1402-1411 (1979)) and its classification was confirmed. The 1H and 13C NMR spectra of fractions #84-45 matched those of the synthesized CA7S, and liquid chromatography-HRMS (LC/HRMS) analysis confirmed that the retention times of fractions #84-45 and the CA7S standard substance were identical. As expected, the synthesized CA7S activated reporter cells expressing MAIT TCR (Fig. 9).

Since the absolute concentration of the unstable 5-OP-RU converted from 5-amino-6-D-ribitylaminouracil (5-A-RU) cannot be accurately estimated, a more stable synthetic ribitylrumazine (RL-7-Me) was used for comparison (ChemBioChem. 22, 672-678 (2021)). The activity of synthetic CA7S was about 1/50 to 1/20 that of RL-7-Me, indicating that CA7S is a weak agonist against MAIT cells (Fig. 10). Synthetic CA7S also increased MR1 molecule expression on the T cell surface (Fig. 11). CA7S activated MAIT TCRs derived from mouse (m) and human (h), regardless of the species of the MR1 molecule (Fig. 12). Therefrom it was revealed that CA7S is a novel ligand for MAIT cells.

Example 3: Evaluation of CA7S analog
(1) CA7S is biosynthesized by sulfation of cholic acid (CA). CA is a major component of bile acids released from the gallbladder into the intestines and helps the absorption of dietary fat through micellation. However, CA (purchased from Nacalai Tesque) itself did not activate the expression of either TCR of MAIT cells or MR1 molecules on the T cell surface (Fig. 13, upper diagram and lower diagram), indicating that the sulfate group is necessary for agonist function.

In addition to the hydroxyl group at position 7 (7-OH) of CA, the 3-OH group and 12-OH group are also potential sulfation sites. Therefore, these isomers were also synthesized according to previous reports (Chem. Pharma. Bull. 27, 1402-1411 (1979)) (sulfated form of 3-OH group: CA3S; sulfated form of 12-OH group: CA12S). While CA3S is present in several mammalian species and CA12S is not, CA3S activated MAIT reporter cells in the presence of MR1 molecules (Fig. 13, upper diagram). Furthermore, all three forms of CA sulfate (CA7S, CA3S, CA12S) similarly increased the expression of the MR1 molecule on the T cell surface (Fig. 13, lower diagram), suggesting that CA sulfate may possess the ability to bind to MR1 molecule.
(2) Since primary bile acids in mice form taurine conjugates (TCAs) in the body, taurine-conjugated CA3S (TCA3S) and taurine-conjugated CA7S (TCA7S) were synthesized according to previous report (Chem. Pharma. Bull. 27, 1402-1411 (1979)), and the influence of C24 modification on the activity was investigated. TCA, TCA3S, and TCA7S showed no activity as either antigens or MR1 molecular ligands (Fig. 13, lower diagram). This suggests that the formation of amino acid conjugates of CA with a hydrophilic group added at position 24 caused the loss of MR binding ability.

Primary bile acids are further dehydrated by symbiotic bacteria to produce secondary bile acids. Sulfate conjugates of secondary bile acids such as deoxy-CA3S (DCA3S), litho-CA3S (LCA3S), and taurolitho-CA3S (TLCA3S) did not show activity in reporter cells (Fig. 13, upper diagram).
(3) In summary, based on the above findings, it was demonstrated that sulfate conjugation of the 3- or 7-OH group of the cholic acid skeleton is important for MAIT cell activation.
*) TCA was purchased from Nacalai Tesque, DCA3S was purchased from Avanti, LCA3S and TLCA3S were purchased from Cayman, and TCA3S and TCA7S were synthesized according to a previous report (Chem. Pharma. Bull. 27, 1402-1411 (1979)).

The structures of CA7S and its analogs evaluated above are shown in Fig. 14.

### Example 4: Analysis of gene expression signature

Human peripheral blood mononuclear cells (PBMCs) were stimulated with CA7S and CA3S for several days, and then sc-TCR-RNA-seq analysis was performed. MAIT cells that survived in the presence of CA7S/3S expressed a representative MAIT TCR (TRAV1-2-TRAJ33/12/20:TRBV6-4/6-1/20-1), which was similar to that observed with 5-OP-RU stimulation (Fig. 15). This suggests that CA7S/3S is a ligand for MAIT cells possessing a representative MAIT TCR. Furthermore, in contrast to 5-OP-RU, CA7S/3S induced genes involved in homeostasis maintenance, such as IL7R, KLF2, and TCF7 (Fig. 16).

Based on the above findings, it was demonstrated that sulfated bile acids are host-derived (endogenous) ligands, different from (exogenous) antigen 5-OP-RU derived from microorganisms, and contribute to the survival and tissue repair of MAIT cells.

### Example 5: Relationship with cholestasis

Bile was collected from healthy and cholestasis-affected individuals, and the concentration of CA3S in the bile (pmol/ml) was measured using a mass spectrometer (LC-MS/MS). The results are shown in Fig. 17.

The CA3S concentration in the cholestasis-affected state increased to about four times that of the healthy state. This indicates that the concentration of CA3S in bile can be used as a diagnostic marker for the onset of cholestasis.

### Example 6: Evaluation of CA7S analog (2)

In the same manner as in Example 3, the activation action of CA7S and analogs different from those evaluated in Example 3 on the TCR of MAIT cells and the activation action on the expression of MR1 molecules on the T cell surface were investigated. Fig. 18A shows the investigation results of the activation action of each analog on TCR of MAIT cells, and Fig. 18B shows the investigation results of the activation action of each analog on the expression of MR1 molecules on the T cell surface.

Analogs 11 and 13 showed activation of the TCR of MAIT cells and activation of the expression of MR1 molecules on the T cell surface, suggesting that these analogs may also function as activation molecules for MAIT cells in vivo.

The structures of the analogs evaluated in this Example are shown below.

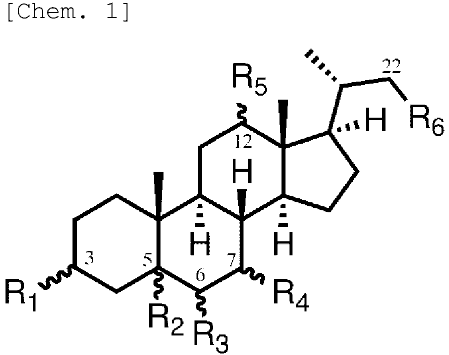

**[Table 1-1]**

| | 10 | 11 | 12 | 13 | 14 | 15 | 16 |
|---|---|---|---|---|---|---|---|
| R₁ | α OSO₃H | oxo | α OH | α OH | α OH | α OH | α OH |
| R₂ | α H | α H | α H | α H | α H | α H | α H |
| R₃ | H | H | H | H | H | H | H |
| R₄ | H | *α* OH | oxo | *α* OH | oxo | α OH | α 0H |
| R₅ | α OH | α OH | α 0H | oxo | oxo | α OH | α OH |
| R₆ | CH₂CO₂H | CH₂CO₂H | CH₂CO₂H | CH₂CO₂H | CH₂CO₂H | C₃H₆CO₂H | CO₂H |

**[Table 1-2]**

| | 17 | 18 | 19 | 20 | 21 | 22 | 23 |
|---|---|---|---|---|---|---|---|
| R₁ | α OH | β OH | α 0H | α OH | α OH | α OH | α OH |
| R₂ | β H | α H | α H | α H | α H | α H | α H |
| R₃ | H | H | H | α OH | β OH | α OH | H |
| R₄ | α OH | α OH | β OH | β OH | α OH | α OH | β OH |
| R₅ | α OH | α OH | α OH | H | H | H | H |
| R₆ | CH₂CO₂H | CH₂CO₂H | CH₂CO₂H | CH₂CO₂H | CH₂CO₂H | CH₂CO₂H | CH₂CO₂H |

**[Table 1-3]**

| | 24 | 25 | 26 |
|---|---|---|---|
| R₁ | α OH | β OH | H |
| R₂ | α H | α H | α H |
| R₃ | H | H | H |
| R₄ | α OH | β OH | α OH |
| R₅ | H | H | α OH |
| R₆ | CH₂CO₂H | CH₂CO₂H | CH₂CO₂H |

### Example 7: Flow cytometry analysis during administration to mouse

The expression of MAIT cells in MR1-5-OP-RU tetramer-enriched thymocytes was analyzed by flow cytometry in 2-4 week old wild-type mice, Sult2a-deficient mice administered with CA7S, and Sult2a-deficient mice not administered with CA7S. The results are shown in Fig. 19 (Figs. A-F).

Fig. A shows a representative flow cytometry dot plot of MR1-5-OP-RU tetramer⁺TCRβ⁺MAIT cells in CD19⁻CD3⁺ cells. Fig. B shows the absolute number of thymic MAIT cells. Fig. C shows representative expression status of CD44 and CD24. Fig. D shows the frequency of MAIT cells in stage 1 (CD44⁻CD24⁺), stage 2 (CD44⁻CD24⁻), and stage 3 (CD44⁺CD24⁻). Fig. E shows the expression of representative CD319 and CD138 in CD44⁺CD24⁻MAIT cells. Fig. F shows the frequencies of MAIT17 cells (CD138⁺) and MAIT1 cells (CD319⁺).

The data were obtained from experiments using three or more mice per group. *P<0.05, **P<0.01, P***<0.005, one-way ANOVA and Tukey's multiple comparison test were used.

The above studies clarified that administration of CA7S to Sult2a-deficient mice restored MAIT cells. This indicates that CA7S is an autoantigen of MAIT cells.

### [Industrial Applicability]

The present invention provides, as embodiments thereof, cells for identifying ligands for MAIT cells, and methods for screening MAIT cell ligands using the cells, and is useful, for example, in the pharmaceutical field.

This application is based on a patent application No. 2023-186971 filed in Japan (filing date: October 31, 2023), the contents of which are incorporated in full herein.

## Claims

1. A T cell that co-expresses an MR1 molecule and a TCR of a MAIT cell and expresses a reporter protein in response to a stimulation of the TCR.

2. The T cell according to claim 1, wherein the reporter protein is selected from green fluorescent protein (GFP), luciferase, and β-glucuronidase.

3. The T cell according to claim 1, wherein the reporter protein is GFP.

4. The T cell according to any one of claims 1 to 3, wherein the MR1 molecule and the TCR of the MAIT cell are derived from human.

5. A method for screening for a MAIT cell agonist or antagonist, comprising at least the following steps:
(1) a step of contacting the T cell according to claim 1 with a test substance, and
(2) a step of observing the activation of a TCR of a MAIT cell and the expression of an MR1 molecule on the T cell surface to
(i) determine a test substance that activates the TCR of the MAIT cell as an agonist of the MAIT cell, and to
(ii) determine a test substance that enhances the expression of the MR1 molecule on the T cell surface without activating the TCR of the MAIT cell as an antagonist of the MAIT cell.

6. The screening method according to claim 5, wherein the activation of the TCR of the MAIT cell is determined by the intensity of GFP emission.

7. The screening method according to claim 5 or 6, wherein the activation of the TCR of the MAIT cell and the expression of the MR1 molecule on the T cell surface are observed by flow cytometry.

8. The screening method according to claim 5 or 6, wherein the test substance is a compound group sample.

9. A biomarker for testing for an inflammatory disease caused by cholestasis, comprising CA3S (cholic acid 3-sulfate) or CA7S (cholic acid 7-sulfate).

10. The biomarker according to claim 9, wherein the inflammatory disease caused by cholestasis is primary biliary cholangitis (PBC) or primary sclerosing cholangitis (PSC).

11. A method for testing for an inflammatory disease caused by cholestasis, comprising measuring a concentration of CA3S or CA7S in bile collected from a test subject.
